# FASCICULE DE BREVET EUROPEEN

(11) **EP 2 512 488 B1**
(45) Date de publication et mention de la délivrance du brevet: **22.04.2015**
(21) Numéro de dépôt: 10799000.4
(22) Date de dépôt: 15.12.2010
(51) Int. Cl.: A61K 31/7004, A61K 36/54, A61P 17/14

(54) **COMPOSITION COMPRENANT AU MOINS UN SUCRE EN C7 POUR LE TRAITEMENT DE L'ALOPÉCIE, POUR LE TRAITEMENT COSMÉTIQUE DES PHANÈRES, ET POUR LE SOIN DES CHEVEUX, CILS OU ONGLES**
ZUSAMMENSETZUNG MIT MINDESTENS EINEM C7-ZUCKER ZUR BEHANDLUNG VON ALOPEZIE, KOSMETISCHE BEHANDLUNG VON HAAR UND NÄGELN SOWIE PFLEGE VON HAAR, WIMPERN ODER NÄGELN
COMPOSITION CONTAINING AT LEAST ONE C7 SUGAR FOR ALOPECIA TREATMENT, COSMETIC TREATMENT OF HAIR AND NAILS, AND CARE OF HAIR, EYELASHES, OR NAILS

(30) Priorité: 16.12.2009 FR 0959075
(43) Date de publication de la demande: 24.10.2012
(73) Titulaire: Laboratoires Expanscience, 92400 Courbevoie (FR)
(72) Inventeur: BAUDOUIN, Caroline, F-78120 Rambouillet (FR); MSIKA, Philippe, F-78000 Versailles (FR)
(74) Mandataire: Regimbeau
(86) Numéro de dépôt international: PCT/EP2010/069806
(87) Numéro de publication internationale: WO 2011/073281

(56) Documents cités:
- WO-A1-2005/115421
- FR-A1- 2 843 125
- PAOLETTI I; BUOMMINO E; TUDISCO L; BAUDOUIN C; MSIKA P; TUFANO M A; BARONI A; DONNARUMMA G: "Patented natural avocado sugars modulate the HBD-2 expression in human keratinocytes through the involvement of protein kinase C and protein tyrosine kinases", ARCHIVES OF DERMATOLOGICAL RESEARCH, vol. 302, no. 3, 16 septembre 2009 (2009-09-16), pages 201-209, XP002579818,

## Description

La présente invention se rapporte à l'utilisation de sucres en C7 dans une composition de soin des phanères, destinée à induire et/ou stimuler leur croissance et/ou à freiner leur chute.

En particulier, l'invention se rapporte à l'utilisation de sucres en C7 pour traiter l'alopécie.

La croissance des cheveux et leur renouvellement sont principalement déterminés par l'activité des follicules pileux et leur environnement matriciel.

Le cheveu est vivant et suit un cycle de croissance naturel : le cycle pilaire. Ce cycle est divisé en trois phases : anagène, catagène et télogène.

La phase anagène est la phase de croissance du cheveu. C'est la partie la plus longue du cycle pilaire puisqu'elle dure de 2 à 5 ans. La très grande majorité des cheveux sont donc en phase anagène.

La phase catagène est une phase de repos pendant laquelle le cheveu cesse d'évoluer. Elle dure environ 3 semaines, ce qui est considérablement peu par rapport à la phase précédente.

La phase télogène, enfin, va aboutir après plus ou moins 3 mois à la mort et à l'expulsion du che anagène. veu, qui va laisser la place à un nouveau follicule en phase

Le nombre de cycles pilaires est quant à lui limité : les cheveux ne connaîtront ainsi que 25 à 30 cycles durant l'ensemble de la vie. Ces cycles ayant une durée de deux à cinq ans, l'homme a théoriquement largement assez de cycles pilaires pour garder une belle chevelure tout au long de sa vie.

Malheureusement, pour des raisons diverses, dont une réaction inflammatoire exacerbée au niveau du follicule pileux, la durée de ces cycles peut considérablement diminuer et conduire à l'épuisement total du potentiel capillaire en seulement quelques années. Les cheveux commencent alors par devenir de plus en plus fins, puis c'est la calvitie qui s'installe.

La chute des cheveux s'accompagne, on le sait maintenant, d'une inflammation au niveau de la racine. L'inflammation conduit à la destruction des follicules pileux et au développement de tissu cicatriciel. Les cytokines pro-inflammatoires ont montré qu'elles étaient capables d'inhiber in vitro la croissance de follicules pileux isolés mis en culture.

L'alopécie est une chute de cheveux sur tout ou partie du cuir chevelu. On perd naturellement en moyenne 60 cheveux par jour. Il existe des variations saisonnières, avec une chute plus importante au printemps et à l'automne. Une chute supérieure à 100 cheveux par jour est toujours excessive. Surtout si elle persiste, la chute devient anormale et doit être traitée.

L'alopécie affecte environ 20% des hommes à partir de 20 ans, puis elle augmente d'environ 10% tous les 10 ans. Chez les hommes, la chute de cheveux se traduit par un dégarnissement des golfes et du sommet du crâne. Elle est progressive et prévisible. Après 50 ans, un peu plus d'un homme sur deux présente un certain degré de calvitie.

Si la calvitie est un phénomène principalement masculin, l'alopécie touche aussi de plus en plus de femmes. On observe chez la femme une diminution globale de la chevelure, prédominante sur le sommet du crâne.

Le terme alopécie recouvre aussi toute une famille d'atteintes du follicule pileux ayant pour conséquence finale la perte définitive, partielle ou générale des cheveux.

On distingue les chutes de cheveux diffuses et les chutes de cheveux localisées. Les chutes de cheveux diffuses : effluvium télogène, effluvium anagène, pelade. Parmi les chutes de cheveux diffuses, les plus fréquentes sont l'alopécie commune (alopécie androgénique masculine et féminine) et l'effluvium télogène (après une fièvre élevée, une grossesse, une prise médicamenteuse ou un régime sévère).

Les chutes de cheveux localisées : alopecie androgénétique, pelade, alopécies cicatricielles, tumeurs. Les chutes de cheveux localisées s'observent dans le cadre de l'alopécie androgénique masculine (golfes, tonsure), de la pelade en plaques, des alopécies induites par des tractions (trichotillomanie, tresses et défrisage) ou des alopécies cicatricielles (alopécie cicatricielle centrale centrifuge, alopécie frontale fibrosante post-ménopausique). Les tumeurs et excroissances de peau s'accompagnent aussi d'une chute de cheveux localisée (hamartome sébacé, carcinome baso-cellulaire, carcinome épidermoïde).

Il semble également que la présence de radicaux libres entretienne l'inflammation des tissus et favorise le vieillissement des cellules du follicule pileux. Cette réaction inflammatoire peut être reconnue comme est une des causes du raccourcissement de la vie des cheveux.

Généralement le phénomène d'alopécie apparaît sur le cuir chevelu mais il peut se manifester également sur le corps entier. Les racines produisent sur les plaques chauves que des cheveux fins qui tombent assez vite. Les racines du cheveu ne meurent pas nécessairement suite à une alopécie. Si l'inflammation disparaît, les cheveux peuvent recommencer à pousser, parfois qu'après des mois, ou même qu'après des années. L'alopécie peut aussi atteindre les ongles.

On recherche depuis de nombreuses années, dans l'industrie cosmétique ou pharmaceutique, des compositions permettant de supprimer ou de réduire l'alopécie, et notamment d'induire ou de stimuler la croissance des fibres kératiniques dont les cheveux ou de diminuer leur chute.

Dans cette optique, on a déjà proposé un grand nombre de compositions comprenant des actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

La demanderesse a trouvé de manière surprenante que des sucres en C7 et dérivés de formule (I), que l'on définira plus loin présentent, entre autre, une activité anti-inflammatoire ayant un effet bénéfique sur la protection des follicules pileux et ainsi sur la protection du follicule après chaque cycle de croissance. Ces sucres en C7 et dérivés sont d'une façon surprenante dotés d'une activité favorable à l'amélioration de la densité des phanères. Ainsi, ces composés ont un effet bénéfique sur la pousse des cheveux humains mais aussi sur la pousse des cils et de certains poils humains ainsi que sur la pousse des ongles.

Le D-mannoheptulose, premier cétoheptose identifié en 1916 par La Forge, de formule générale (II) 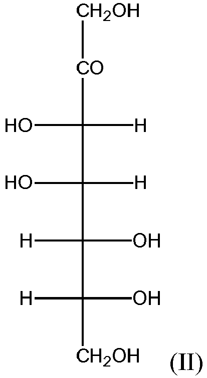 se retrouve dans certaines plantes, en particulier dans la luzerne (*Medicago sativa* L.), dans l'avocat, dans la figue (*Ficus officinalis* L.) dans l'orpin (*Sedum spectabile Bor*.) et dans la primevère (*Primula officinalis* Jacq.). Toutefois, c'est dans l'avocat, que l'on retrouve les teneurs les plus importantes en D-mannoheptulose. Le D-mannoheptulose a déjà été utilisé dans des applications thérapeutiques. Par exemple, la demande de brevet WO95/03809 décrit l'utilisation du D-mannoheptulose, en tant qu'inhibiteur de glucokinase, pour inhiber le développement des cellules tumorales et la demande US2003/0092669 décrit un complément alimentaire oral comprenant du D-mannoheptulose, qui permet de diminuer le taux d'insuline et qui permet ainsi une perte de poids.

Le perséitol, forme polyol du D-mannoheptulose, de formule générale (III) 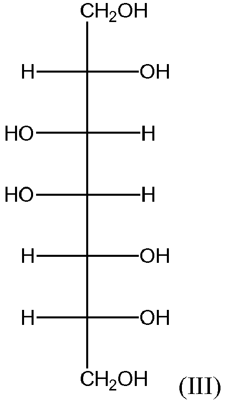 se retrouve également dans l'avocat, en particulier dans le fruit ou dans le noyau de l'avocat.

D'après la publication «Search for pharmacochemical leads from tropical rainforest plants », Hitotaka Shibuya et al. Pure Appl. Chem., vol. 71, n°6, pp 1109-1113, 1999, le perséitol, associé à un ion potassium, permet d'inhiber l'incorporation de leucine-3H dans des cellules tumorales d'ascite sarcomateuse d'Ehrlich.

L'utilisation de ces sucres (perséitol et D-mannoheptulose) pour stimuler la synthèse des beta-défensines humaines (en particulier HBD-2) a déjà été décrite (WO 2005/115421). En particulier, il a été montré dans cette demande que les sucres d'avocat induisent la synthèse de HBD-2 sans induire la synthèse de médiateurs inflammatoires (ce qui signifie que les sucres d'avocat ne sont pas capables de stimuler la réaction inflammatoire mais ce qui ne permet pas de préjuger d'une quelconque action anti inflammatoire). L'utilisation de ces sucres dans le traitement de candidoses et de pityrosporoses a également déjà été décrite (WO 2008/025847).

L'invention a pour objet une composition comprenant au moins un sucre en C7 ou dérivé de formule (I) suivante 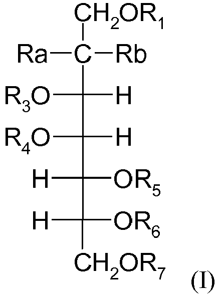 dans laquelle
Ra représente un atome d'hydrogène et Rb représente un -OR₂ ou CRaRb représente le radical CO ;
R₁, R₂, R₃, R₄, R₅, R₆ et R₇ représentent, indépendamment l'un de l'autre
   - un atome d'hydrogène ou
   - un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique ; ou
   - un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique ;
et un excipient pharmaceutiquement acceptable pour le traitement de l'alopécie.

Le terme « alopécie » désigne aussi bien les chutes de cheveux diffuses que les chutes de cheveux localisées. Il recouvre en particulier la pelade ou alopécie areata, la chute diffuse, l'alopécie cicatricielle, l'alopécie non cicatricielle et la maladie de Quinquaud.

La chute diffuse est caractérisée par des cheveux fins et clairsemés sur l'ensemble du cuir chevelu. Elle est souvent due à des facteurs externes tels que : le stress, la fièvre élevée, la fatigue, le surmenage, la prise de médicaments, les déséquilibres alimentaires, les suites d'accouchements, les interventions importantes, certaines maladies infectieuses ou tumeurs, la pollution... Elle est en très forte augmentation, elle toucherait entre 20 et 40 % des femmes.

L'alopécie areata ou pelade correspond à des plaques d'alopécie dues à une inflammation de la racine des cheveux, suite à une réaction d'auto défense du système immunitaire.

L'alopécie cicatricielle correspond à une chute des cheveux due à une maladie inflammatoire de la peau (infection, inflammation, tumeur, blessure, brûlure...) qui détruit définitivement le bulbe pilaire.

L'alopécie non-cicatricielle correspond elle à une chute des cheveux due à un dysfonctionnement du follicule pileux, qui provoque une interruption prématurée de la phase de croissance.

La maladie de Quinquaud est une maladie orpheline qui se caractérise par l'inflammation des follicules qui se trouvent à la racine des cheveux et des poils. L'inflammation de ces follicules provoque la perte des cheveux (alopécie), qui tombent alors en plaques. C'est une maladie extrêmement handicapante et très pénible à assumer.

La Demanderesse a ainsi constaté que les sucres en C7 trouvés dans l'avocat, le perséitol et le mannoheptulose, ainsi que leurs dérivés résultant de l'estérification d'une ou plusieurs des fonctions hydroxyles du sucre avantageusement avec un acide gras, sont efficaces dans le traitement de l'alopécie et permettent de stimuler la pousse des phanères.

On entend par "phanères", les cheveux, les cils, les sourcils, les ongles et notamment les cheveux et les ongles.

Selon une première variante avantageuse de l'invention, les sucres en C7 sont sous une forme libre (les fonctions hydroxyles ne sont pas estérifiées). La composition comprend donc un sucre en C7 choisi dans le groupe constitué par le mannoheptulose, le perséitol et leurs mélanges.

La source de D-mannoheptulose et/ou de perséitol peut être un extrait hydrosoluble de sucres d'avocat ou d'une autre plante. Autrement, le D-mannoheptulose et le perséitol sont disponibles commercialement (origine synthétique). Selon une variante avantageuse de l'invention, la source de D-mannoheptulose et/ou de perséitol est un extrait hydrosoluble de sucres d'avocat.

L'extrait hydrosoluble de sucres d'avocat peut directement être obtenu à partir de n'importe quelle partie de l'avocat ou de l'avocatier, telle que le fruit, la peau, le noyau, la feuille ou les racines de l'avocatier. Il est aussi possible d'obtenir un extrait peptidique d'avocat à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile (extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. L'extrait est avantageusement obtenu à partir du fruit frais de l'avocatier. Les fruits pourront être choisis parmi les variétés *Hass, Fuerte*, *Ettinger, Bacon, Nabal, Anaheim*, *Lula, Reed, Zutano*, *Queen, Criola Selva, Mexicana Canta, Region Dschang, Hall, Booth, Peterson, Collinson Redn,* plus avantageusement parmi les variétés *Hass, Fuerte et Reed.* De préférence, on retiendra les variétés *Hass, Fuerte*, *Ettinger et Bacon,* et plus avantageusement les variétés *Hass* et *Fuerte*.

Le fruit de l'avocatier est principalement constitué d'eau, de pulpe, d'huile et de noyau. Les proportions de ces constituants sont, à l'instar de toutes matières naturelles et végétales, extrêmement variables. Toutefois, on admet généralement les données de composition moyennes suivantes, exprimées en pourcentage de fruit frais, données dans le tableau 1 suivant :

**Tableau 1**

| | |
|---|---|
| Eau | 70-85 % |
| Protéines | 1,5-4,5 % |
| Lipides | 12-23 % |
| Sucres | 1,5-5 % |
| Fibres | 1,1-1,6 % |

De fait, l'avocat n'est pas particulièrement riche en polysaccharides. Cependant, la nature des monosaccharides solubles est tout à fait particulière, tels que le perséitol ou le D-mannoheptulose constitués de 7 atomes de carbone.

L'extrait hydrosoluble de sucres d'avocat est susceptible d'être obtenu par un procédé comprenant les étapes successives suivantes :
- obtention d'un tourteau d'avocat, avantageusement du fruit de l'avocat, par séchage de l'avocat puis extraction des lipides (huile) ; ensuite
- délipidation complète dudit tourteau, puis lavage à l'eau ou à un milieu hydroalcoolique puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- le cas échéant, concentration sous vide et conditionnement.

La première étape du procédé consiste à sécher le fruit puis à le déshuiler. Ainsi, après tranchage du fruit en fines lamelles, son séchage peut être réalisé par l'ensemble des techniques connues de l'homme de métier, parmi lesquelles on peut citer le séchage à l'air chaud, la lyophilisation ou encore le séchage osmotique. D'une façon générale, la température lors de cette étape de séchage sera avantageusement maintenue, quelle que soit la technique employée, inférieure ou égale à 80°C. Dans le cadre du présent procédé, pour des raisons de facilité de mise en oeuvre et pour des raisons de coût, le séchage en séchoirs ventilés, en couche mince et sous courant d'air chaud, à une température comprise entre 70 et 75°C est préféré. La durée de l'opération peut varier de 5 à 72 heures.

Les lipides du fruit séché sont par la suite extraits soit par voie mécanique dans une presse à vis continue, ou encore par voie chimique, à l'aide d'un solvant tel que l'hexane, dans un extracteur de type soxhlet ou dans un extracteur continu à bande, de type De Smet®, notamment selon le procédé décrit dans la demande FR 2 843 027, ou par un procédé utilisant le CO₂ supercritique. Parmi les intérêts majeurs du procédé, l'huile co-produite constitue un produit bien évidemment directement valorisable. C'est pourquoi, on préfère l'extraction des lipides par voie mécanique. Le fruit sec et déshuilé, encore appelé tourteau, peut subir ensuite les étapes suivantes :
- délipidation complète, notamment à l'acétone et/ou à l'éthanol,
- décantation puis lavage du tourteau à l'eau et/ou un mélange hydroalcoolique,
- centrifugation, filtration, récupération de la fraction soluble (élimination du gâteau),
- concentration,
- déminéralisation par échange d'ions
- ultrafiltration avec un seuil de coupure de 10 kDa,
- concentration sous vide, ajout de conservateur et conditionnement.

De façon générale, l'extrait aqueux final peut contenir en poids 0,1 à 20 % de matière sèche, avantageusement 1 à 10 % de matière sèche, encore plus avantageusement 3 à 5 % de matière sèche. La teneur en sucres en C7, c'est-à-dire en D-mannoheptulose et en perséitol, dans la matière sèche est avantageusement comprise entre 50 et 100 %, plus particulièrement entre 65 et 90% en poids, par rapport au poids total de la matière sèche. Les données analytiques moyennes d'une solution aqueuse à 5 % d'extrait sec, obtenue par le procédé décrit précédemment, sont données dans le tableau 2 suivant :

**Tableau 2**

| | | |
|---|---|---|
| pH (dilution ¼) | | 3-5 |
| Absorbance (dilution ½) | 420 nm | Inférieure à 0,200 |
| | 550 nm | Inférieure à 0,050 |
| Sucres en C7 / matière sèche | | 50 - 100 % |

La composition relative en sucres de l'extrait hydrosoluble, en poids par rapport au poids total de la matière sèche de l'extrait, répond avantageusement aux critères suivants (composition relative déterminée par HPLC ; high performance liquid chromatography = chromatographie liquide haute performance):
- D-mannoheptulose 0 à 100%, en particulier 5 à 80 %,
- Perséitol 0 à 100%, en particulier 5 à 80 %,
- Saccharose inférieur à 10%,
- Glucose inférieur à 10%,
- Fructose inférieur à 10%.

L'extrait hydrosoluble de sucre d'avocat comprend avantageusement, par rapport au poids total de la matière sèche, 10 à 80% en poids de D-mannoheptulose plus avantageusement 15 à 70 % en poids de D-mannoheptulose. L'extrait hydrosoluble de sucres d'avocat comprend avantageusement, par rapport au poids total de la matière sèche, 20 à 80% en poids de perséitol, plus avantageusement 25 à 70% en poids de perséitol.

De préférence, la composition relative en sucres de l'extrait hydrosoluble, en poids par rapport au poids total de la matière sèche de l'extrait, répond aux critères suivants (composition relative déterminée par HPLC) :
- D-mannoheptulose 25 à 60 %,
- Perséitol 25 à 60 %,
- Saccharose inférieur à 10 %,
- Glucose inférieur à 10 %,
- Fructose inférieur à 10 %.

D'une manière surprenante les inventeurs ont constaté un effet de synergie entre le D-mannoheptulose et/ou le perséitol et les sucres minoritaires (fructose, glucose, saccharose) présents dans l'extrait de sucres d'avocat.

L'extrait obtenu pourra éventuellement être lyophilisé dans le but d'obtenir une poudre solide (extrait sec), totalement hydrosoluble.

L'extrait obtenu pourra éventuellement être fractionné en chaque sucre purifié. Cette séparation et purification peut être réalisée par l'ensemble des techniques connues de l'homme de métier, parmi lesquelles on peut citer de façon non exhaustive la précipitation/filtration, la recristallisation, ou encore la séparation par chromatographie telle que le procédé I.S.M.B. (Improved Simulated Moving Bed).

Selon une deuxième variante avantageuse de l'invention, les sucres en C7, que sont avantageusement le D-mannoheptulose et le perséitol, sont au moins partiellement estérifiés avec un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique. En particulier les sucres en C7 sont au moins partiellement estérifiés avec un résidu d'acide gras. La chaine hydrocarbonée peut être linéaire ou ramifiée, elle est avantageusement linéaire.

Le radical R représente avantageusement un résidu d'un acide gras.

Les acides gras considérés selon l'invention sont plus particulièrement des acides gras à longue chaîne, c'est-à-dire pouvant posséder plus de 11 atomes de carbone et notamment plus de 14 atomes de carbone.

Leur chaine hydrocarbonée peut être saturée ou contenir une ou plusieurs doubles liaisons. A titre représentatif de ces acides gras, on peut notamment citer les acides gras saturés comme les acides palmitique (C₁₆), stéarique (C₁₈), arachidique (C₂₀), béhénique (C₂₂) et lignocérique (C₂₄) et les acides gras insaturés comme les acides palmitoléique (C₁₆), oléique (C₁₈), linoléique (C₁₈), linolénique notamment sous ses formes α et γ (C18) et arachidonique (C₂₀).

En particulier le radical R est avantageusement choisi dans le groupe constitué par un radical stéaryle, linoléyle, oléyle, palmityle, lauryle, myristyle, arachidyle, béhényle, lauroléyle, myristoléyle, palmitoléyle, linolényle sous ses formes α et γ, et/ou arachidonyle.

Il est particulièrement avantageux de substituer la chaîne hydrocarbonée avec groupement -SO₃M avec M représentant un atome d'hydrogène, un ion ammonium NH₄⁺ ou un ion métallique (en particulier sodium).

Dans les dérivés de formule (I), les fonctions hydroxyles peuvent être substituées par le résidu du même acide gras ou par des résidus d'acides gras différents.

Les dérivés d'acides gras des sucres en C7 peuvent être obtenus par réaction d'estérification résultant de la mise en contact, dans les conditions appropriées, d'un ou plusieurs acides de formule HOOC-R (R ayant la même définition que dans les paragraphes précédents) avec le D-mannoheptulose et/ou le perséitol disponibles commercialement (origine synthétique) ou avec l'extrait hydrosoluble de sucres d'avocat décrit ci-dessus.

Selon une troisième variante avantageuse de l'invention, les sucres en C7, que sont avantageusement le D-mannoheptulose et le perséitol, sont au moins partiellement estérifiés avec un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique. La chaine hydrocarbonée peut être linéaire ou ramifiée, elle est avantageusement linéaire.

Le radical R représente avantageusement un résidu d'un acide à courte chaîne, c'est-à-dire pouvant posséder moins de 10 atomes de carbone et notamment moins de 8 atomes de carbone.

Leur chaine hydrocarbonée peut être saturée ou contenir une ou plusieurs doubles liaisons. A titre représentatif de ces acides, on peut notamment citer l'acide acétique.

Il est particulièrement avantageux de substituer la chaîne hydrocarbonée avec groupement -SO₃M avec M représentant un atome d'hydrogène, un ion ammonium NH₄⁺ ou un ion métallique (en particulier sodium).

Dans les dérivés de formule (I), les fonctions hydroxyles peuvent être substituées par le résidu du même acide ou par des résidus d'acides différents.

Les dérivés d'acides des sucres en C7 peuvent être obtenus par réaction d'estérification résultant de la mise en contact, dans les conditions appropriées, d'un ou plusieurs acides de formule HOOC-R' (R' ayant la même définition que dans les paragraphes précédents) avec le D-mannoheptulose et/ou le perséitol disponibles commercialement (origine synthétique) ou avec l'extrait hydrosoluble de sucres d'avocat décrit ci-dessus.

Selon une quatrième variante avantageuse de l'invention, les sucres en C7, que sont avantageusement le D-mannoheptulose et le perséitol, sont au moins partiellement estérifiés avec un radical -(CO)-R et avec un radical -(CO)-R', R et R' ayant les mêmes définitions que celles données dans les deuxième et troisième variantes.

Les dérivés d'acides des sucres en C7 peuvent être obtenus par réaction d'estérification résultant de la mise en contact, dans les conditions appropriées, d'un ou plusieurs acides de formule HOOC-R et d'un ou plusieurs acides de formule HOOC-R' avec le D-mannoheptulose et/ou le perséitol disponibles commercialement (origine synthétique) ou avec l'extrait hydrosoluble de sucres d'avocat décrit ci-dessus.

On appelle les dérivés obtenus par les deuxième, troisième ou quatrième variantes dérivé d'acide de D-mannoheptulose ou respectivement de perséitol.

Dans l'une quelconque des deuxième, troisième ou quatrième variantes, le rapport entre le nombre de fonctions esters du composé de formule (I) et le nombre de fonctions hydroxyles initiales, ou taux d'estérification, pour une molécule de sucre, varie de 0,2 à 1. Il est notamment inférieur ou égal à 0,6, et en particulier inférieur ou égal à 0,4.

Le degré d'estérification est contrôlé par la concentration des réactifs, la durée de réaction et la température de réaction. Il peut être mesuré par chromatographie, en particulier par chromatographie par exclusion stérique.

Selon l'une ou l'autre des quatre variantes, la composition comprend 0,001 à 30 % en poids de D-mannoheptulose ou de son dérivé d'acide, par rapport au poids total de ladite composition, et/ ou 0,001 à 30 % en poids de perséitol ou de son dérivé d'acide, par rapport au poids total de ladite composition. Plus particulièrement, la composition comprend 0,002 à 5 % en poids de D-mannoheptulose ou de son dérivé d'acide, par rapport au poids total de ladite composition, et/ ou 0,002 à 5 % en poids de perséitol ou de son dérivé d'acide, par rapport au poids total de ladite composition.

Dans le cadre de la présente invention, la composition peut en outre comprendre
- un autre actif pour traiter l'alopécie ; et/ou
- un agent anti-chute des cheveux et/ou fortifiant pour le cheveu et les ongles ; et/ou
- un agent antipelliculaire.

A titre d'agent pour traiter l'alopécie, on peut notamment citer les dérivés de DHEA (7-hydroxy DHEA et 7 céto-DHEA), la taurine et ses dérivés (EP1 515 712), les inhibiteurs de la 15-hydroxy prostaglandine déshydrogénase (tels que les composés tétrazoliques et autres composés décrits dans EP 1 358 868), les agonistes des récepteurs à l'acide rétinoïque (tels que les composés décrits dans EP 829 256), les dérivés de la 4-aminopiperidine (EP1 849 455, EP 1 849 456), les dérivés de N-oxyde (EP1829523), les dérivés de thiazolidine-2,4-dione (EP1775294, EP1739083, EP1738742), les dérivés 2-thioacétamide (EP1052576), les dérivés styryl-pyrazole (EP 1 558 203), les dérivés d'acide pyridine-dicarboxylique (EP 1 352 629), les dérivés indolecarboxyliques (Ep 964 852), les dérivés 2-amino-alkane-1,3- diol (EP 790053), du fructose, du glucose et/ou des protéines globulaires de céréales ou leur hydolysats (EP648107) et les dérivés de pyrimidine (EP 522 964, EP 540 629, EP 459 890, EP 420 707, EP 376 821, EP 357 484, EP 347 328, EP 336 813, EP 336 812, EP 321 951, EP 319 027, EP 304 665, EP 2 119 475 - 2,4-diaminopyrimidine-3-N-oxyde-).

Les agents anti-chute des cheveux et/ou fortifiant pour le cheveu et les ongles sont avantageusement les phytostérols, les isoflavones comme par exemple les isoflavones de soja, le RTH16®, l'aminexil®, le minoxidil®, le viviscal® le rétinol, le zinc et ses dérivés, la neoruscine, la vitamine E, la vitamine B2, la vitamine B3, la vitamine B6, la vitamine PP, la vitamine B5 (panthénol, bépanthène, dexpanthénol), la vitamine B8 (vitamine H ou biotine), la vitamine B9 (acide folique), l'alpha hydroxyacide, la quinine, certains acides aminés soufrés comme la cystéine, la cystine, la méthionone. On peut également citer les inhibiteurs de 5-alpha réductase tels que par exemple le finastéride, le dutastéride, *serenoa serrulata* ou *repens*, l'extrait de *Cucurbita pepo* ou encore certains phyostérols. On peut également citer la kératine, les oligoéléments, les sels minéraux. On peut également utiliser des extraits protéiques ou lipidiques végétaux comme par exemple les extraits de pfaffia, de sauge, de citron, de ginseng, de quinquina, de jojoba, de marron d'inde, de miel, de blé, d'ortie, d'échinea, de cophra, de noix de coco.

Les agents anti-pelliculaires (cuir chevelu) sont avantageusement l'extrait de capucine, la vitamine F, le thymol, l'argile, le pyrithione de zinc, le zinc-PCA, le gluconate de zinc, le sulfate de zinc, le camphre, l'extrait de myrte, l'acide salicylique, la vitamine B5, le climbazole, l'ichtyol, le sélénium et ses dérivés, l'extrait de Curbicia, l'extrait de Carthame, l'extrait d'huile de Melaleuca, l'huile de Bourrache et de Mimosa Tenuiflora, la Propolis, le Kertyol, l'acide glycolique, le kéluamide, la cyclopiroxolamine, le piroctone olamine, le capryloyl glycine.

Dans le cadre de la présente invention, la composition peut également en outre comprendre un actif dermatologique choisi dans le groupe constitué par les actifs hydratants, des activateurs de la synthèse de kératine, les kératorégulateurs, les kératolytiques, les agents restructurant de la barrière cutanée (activateurs de la synthèse des lipides cutanés ou activateurs de la différenciation), les agents cicatrisants, les agents sébo-régulateurs, les agents anti-irritants, les agents apaisants, les agents anti-inflammatoires, les agents anti-oxydants, les agents anti-âge, et leurs mélanges.

Les actifs hydratants les plus utilisés sont la glycérine ou ses dérivés, l'urée, l'acide pyrrolidone carboxylique et ses dérivés, l'acide hyaluronique de tout poids moléculaire, les glycosaminoglycanes et tout autres polysaccharides d'origine marine, végétale ou biotechnologique comme par exemple la gomme xanthane, le fucogel®, certains acides gras comme l'acide laurique, l'acide myristique, les acides gras poly- et mono-insaturés de type oméga 3, 6 et 7, 9 comme l'acide linoléique et acide palmitoléique, l'oléodistillat de tournesol, les peptides d'avocat, le beurre de cupuaçu.

Les activateurs de la synthèse de kératine pouvant être utilisés en association sont avantageusement les rétinoïdes, les peptides de lupin, des protéines clés du *stratum corneum* ou *granulosum* (kératines) et des cornéodesmosomes, les peptides de quinoa.

Parmi les agents kératorégulateurs/kératolytiques les plus utilisés se trouvent : les acides de fruits alpha hydroxyacide - AHA (acide citrique, acide glycolique, acide malique, acide lactique...), les esters d'AHA, les associations d'AHA avec d'autres molécules comme l'association acide malique et protéines d'amandes (keratolite®), l'association acide glycolique ou acide lactique avec l'arginine ou encore l'association d'hydoxy-acide avec des molécules lipidiques comme le LHA® pour lipo-hydroxyacide, les complexes d'hydroxyacide amphotères - AHCare, l'ecorce de saule (Salix alba bark extract), l'acide azélaïque et ses sels et esters, l'acide salicylique et ses dérivés comme l'acide capriloyl salicylique ou en association avec d'autres molécules comme l'association acide salicylique et polysaccharide (beta hydroxyacide - BHA), tazarotène, adapalène, ainsi que les molécules de la famille des rétinoïdes tels que : trétinoïne, rétinaldéhyde, isotrétinoïne, rétinol.

Les agents cicatrisants et restructurant pouvant être utilisés en association sont avantageusement la vitamine A, le panthénol (vitamine B5), le lupéol, l'extrait peptidique de maca, un extrait de quinoa, l'arabinogalactane, l'oxyde de zinc, le magnésium, le silicium, l'acide madécassique ou asiatique, le sulfate de dextran, le coenzyme Q10, la glucosamine et ses dérivés, la chondroïtine sulfate et globalement les glucosaminoglycanes ou GAG, le sulfate de dextran, les céramides, le cholestérol, le squalane, les phospholipides, les peptides de soja fermentés ou non, les peptides végétaux, les polysaccharides marins, végétaux ou biotechnologiques comme les extraits d'algues ou l'extrait de fougère, les oligo-éléments, les extraits de plantes à tanin comme les tanins dérivant de l'acide gallique appelés tanins galliques ou encore hydrolysables, initialement trouvé dans la noix de galle, et les tanins catéchiques résultant de la polymérisation d'unités flavaniques dont le modèle est fourni par le Cachou (*Acacia catechu*). Les oligo-éléments pouvant être utilisés sont avantageusement choisis dans le groupe constitué par le cuivre, le magnésium, le manganèse, le chrome, le sélénium, le silicium, le zinc et leurs mélanges. Peuvent également être utilisés des concentrats de tournesol, plus avantageusement des concentrats de tournesol linoléiques, tels que l'actif commercialisé par les Laboratoires Expanscience, Soline®, des insaponifiables d'huile végétale, tel que l'Avocadofurane®, des agonistes PPARs (rosiglitazone, pioglitazone), RXR, LXR.

Les agents sébo-régulateurs pouvant être utilisés en association sont avantageusement choisis dans le groupe constitué par les inhibiteurs de 5-alpha réductase comme par exemple l'actif 5-alpha Avocuta®. Le zinc (et ses sels gluconate, salicylate et acide pyroglutamique) présente aussi une activité sébo-suppresseur. On peut citer aussi la spironolactone, anti-androgène et antagoniste de l'aldostérone, qui engendre une réduction significative du taux de sécrétion de sébum après douze semaines d'application. D'autres molécules telles que par exemple *Cucurbita pepo,* extraite des graines de citrouille, et l'huile de pépins de courge, le sabal, limitent la production de sébum par inhibition de la transcription et de l'activité de la 5α-réductase. D'autres séborégulateurs d'origine lipidique agissant sur la qualité du sébum, comme l'acide linoléique présentent un intérêt.

Les agents anti-inflammatoires / anti-irritants, apaisants limitent la réaction inflammatoire conduite *via* les cytokines ou médiateurs du métabolisme de l'acide arachidonique et ont des propriétés apaisantes et anti-irritantes. Les plus classiques sont l'acide glycyrrhétinique (les dérivés de réglisse) avec ses sels et esters, l'acide lipoïque, le beta-carotène, la vitamine B3 (niacinamide, nicotinamide), la vitamine E, la vitamine C, la vitamine B12, les flavonoïdes (thé vert, quercétine...), le lycopène ou la lutéine, l'oléodistillat d'avocat, l'arabinogalactane, les peptides de lupin, un extrait total de lupin, un extrait de quinoa, le Cyclocéramide® (dérivé d'oxazoline, produit appelé OX100 dans la demande WO 03/055463), les isoflavones comme par exemple la génistéine/génistine, daidzéine/daidzine, les eaux de source ou thermales (eau d'Avène, eau de la Roche Posay, eau de Saint Gervais, eau d'Uriage, eau de Gamarde), les extraits de Goji (*Lycium barbarum*), les peptides ou complexes d'acides aminés végétaux ou encore la disulone topique, ou les médicaments anti-inflammatoires stéroïdiens (AIS), tels que les corticoïdes, ou non-stéroïdiens (AINS).

On entend par agent antioxydant, une molécule qui diminue ou empêche l'oxydation d'autres substances chimiques. Les antioxydants pouvant être utilisés en association sont avantageusement choisis dans le groupe constitué par les thiols et les phénols, par les dérivés de réglisse comme l'acide glycyrrhétinique avec ses sels et esters, l'alpha bisabolol, l'extrait de ginkgo biloba, de calendula, le Cyclocéramide® (dérivé d'oxazoline), les peptides d'avocat, les oligo-éléments comme le cuivre, le zinc, et le sélénium, l'acide lipoïque, la vitamine B12, la vitamine B3 (niacinamide, nicotinamide), les vitamines C, les vitamines E, le co-enzyme Q10, le krill, le glutathion, le BHT pour butylhydroxytoluène, le BHA pour butylhydroxyanisol, le lycopène ou la lutéine, le beta-carotène, la grande famille des polyphénols comme les tanins, les acides phénoliques, les anthocyanes, les flavonoïdes avec par exemple les extraits de thé vert, de fruits rouges, de cacao, de raisin, de *Passiflora incarnata*, de *Citrus* ou encore les isoflavones comme par exemple la génistéine/génistine, daidzéine/daidzine, Dans le groupe des anti-oxydants on trouve aussi les substances anti-glycation telles que la carnosine ou certains peptides, la n-acetyl-cystéine, ainsi que les enzymes anti-oxydants ou radicalaires comme la SOD (super oxyde dismutase), la catalase, la glutathion peroxydase, la thioredoxine reductase et leurs agonistes.

Les agents anti-âge sont avantageusement des agents anti-oxydants et en particulier la vitamine C, ou encore la vitamine A, le rétinol, le rétinal, l'acide hyaluronique de tout poids moléculaire, l'Avocadofurane®, les peptides de lupin, l'extrait peptidique de maca.

Dans le cadre de la présente invention, la composition peut également en outre comprendre un actif choisi dans le groupe constitué par les pré et probiotiques, les agents anti-bactériens, les composés antifongiques, les conservateurs, les immunomodulateurs, les facteurs de croissance, les filtres et écrans solaires minéraux ou organiques (pigmentaires ou ultrafins),

Les pré et probiotiques pouvant être utilisés en association sont avantageusement les trans-galacto-oligosaccharides, les fructanes ou les fructo-oligosides pour les prébiotiques et les probiotiques appartenant aux genres *Lactobacilli* et *Bifidobacteria*.

Les composés antifongiques pouvant être utilisés en association sont avantageusement l'econazole et le ketoconazole.

Les conservateurs et agents antibactériens pouvant être utilisés en association sont par exemple ceux généralement utilisés en cosmétique ou en nutraceutique, les molécules à activité anti-bactérienne (pseudo-conservateurs) tels que les dérivés capryliques comme par exemple le capryloyl glycine et le glyceryl caprylate, tels que l'hexanediol, et le sodium levulinate, les dérivés de zinc et de cuivre (gluconate et PCA), la phytosphingosine et ses dérivés, le peroxyde de benzoyle, la piroctone olamine, le pyrithione zinc, le sulfure de sélénium. Les conservateurs antiseptiques pouvant être utilisés en association sont par exemple le triclosan, la chlorhéxidine, les ammoniums quaternaires.

Les immunomodulateurs pouvant être utilisés en association sont par exemple le tacrolimus, le pimécrolimus et les oxazolines. Les oxazolines pouvant être utilisées en association sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide^{®}.

Les facteurs de croissance pouvant être utilisés en association sont avantageusement la becaplermine et le TGF-beta (Transforming Growth Factor beta), l'EGF, le NGF, le VEGF.

A titre d'exemple d'actifs protecteur solaire, on peut notamment citer le dioxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S), l'octocrylène, ,le butyl méthoxydibenzoylméthane, acide terephthalylidene dicamphor sulfonique, 4-méthylbenzylidène de camphre, benzophénone, ethylhexyl méthoxycinnamate, éthylhexyl diméthyl PABA, diéthylhexyl butamido triazone.

Dans le cadre de la présente invention, la composition peut également en outre comprendre un ou plusieurs (en particulier deux, trois ou quatre) actifs choisi dans le groupe constitué par :
- un extrait de fruit de *Schizandra sphenanthera*, en particulier un extrait peptidique et osidique de *Schizandra sphenanthera* ;
- un extrait peptidique d'avocat, en particulier celui décrit dans la demande WO2005/105123 ;
- une huile d'avocat (voir les demandes internationales WO2004/012496, WO2004/012752, WO2004/016106, WO2007/057439) ;
- des furanes d'avocat, en particulier l'Avocadofurane® (furanes d'avocat, pouvant être obtenus par le procédé décrit dans la demande internationale WO 01/21605) ;
- un ester gras dont la chaîne grasse est une chaîne hydrocarbonée linéaire en C7-C30 comportant entre 0 et 2 insaturations éthyléniques et pouvant être substituée par 1 à 3 groupe hydroxy et/ou 1 à 3 fonctions esters en plus de la fonction ester principale, en particulier le 5 alpha avocuta® (butyl avocadate), pour inhiber la 5 alpha réductase (voir WO 01/52837 et WO 02/06205) ;
- des insaponifiables d'avocat et/ou de soja, avantageusement un mélange d'insaponifiables d'avocat furanique et d'insaponifiables de soja, dans un rapport respectif d'environ 1/3-2/3. Les insaponifiables d'avocat et de soja sont encore plus avantageusement le produit Piasclédine®, commercialisé par les Laboratoires Expanscience. ;
- un oléodistillat de tournesol, encore plus avantageusement avec des concentrats de tournesol linoléiques, tels que l'actif commercialisé par les Laboratoires Expanscience, Soline® (cf. la demande internationale WO 01/21150) ;
- du lupéol (FR 2 822 821, FR 2 857 596) ;
- des peptides de lupin tels qu'obtenues selon le procédé décrit dans la demande WO2005/102259. ;
- un extrait total de lupin (voir demande internationale WO2005/102259) ;
- une huile de lupin, avantageusement une huile de Lupin blanc doux, telle que celle décrite dans la demande internationale WO 98/47479. ;
- un extrait peptidique de maca (voir demande internationale WO2004/112742) ;
- une oxazoline, en particulier la 2-undécyl-4,4-diméthyl-1,3-oxazoline (Cyclocéramide®) tel que décrit dans les demandes internationales WO2004050052, WO2004050079 ;
- des peptides de riz tels que décrits dans la demande internationale WO 2008/009709) ;
- un extrait de quinoa, en particulier un extrait peptidique de quinoa (WO2008/080974) ;
- du beurre de Cupuaçu ;
- un concentrat de colza ou de maïs.

La composition est avantageusement destinée à une administration topique ou par voie orale.

Selon une variante avantageuse, les compositions selon l'invention sont adaptées à l'administration topique sur le cuir chevelu et incluent les shampooings, les gels, les émulsions, les laits, les lotions, les huiles, les solutions aqueuses ou hydro -alcooliques ou glycoliques, les poudres, les sprays ou tout autre produit pour application externe, comme par exemple les vernis pour une application sur les ongles.

L'invention a également pour objet un procédé de traitement cosmétique des phanères, en particulier des cheveux, destiné à stimuler leur croissance et/ou freiner leur chute, caractérisé en ce qu'il consiste à administrer une composition cosmétique comprenant au moins un dérivé de formule (I) tel que défini selon l'une quelconque des quatre variantes, éventuellement en association avec un ou plusieurs des actifs cités précédemment.

L'invention a également pour objet un procédé de soin cosmétique des cheveux et/ou cils et/ou ongles, en vue d'améliorer leur état et/ou leur aspect, caractérisé en ce qu'il consiste à administrer une composition cosmétique comprenant au moins un dérivé de formule (I) tel que défini selon l'une quelconque des quatre variantes, éventuellement en association avec un ou plusieurs des actifs cités précédemment.

Selon ce procédé de soin cosmétique, la composition cosmétique est avantageusement appliquée sur les cheveux et/ou cils et/ou ongles puis laissée au contact des cheveux et/ou cils et/ou ongles et éventuellement rincée.

Selon ce procédé de soin cosmétique, la composition cosmétique est avantageusement administrée par voie orale, de préférence sous la forme de capsules, de gélules ou de comprimés ou encore de barres céréalières ou boissons. Les doses minimales et maximales par jour sont avantageusement comprises entre 10 et 250mg selon l'indication : pour un traitement préventif, on recommande une prise 1 à 2 fois/jour pendant 2 mois (soit 10 à 150mg/jour) et pour un traitement curatif, on recommande une prise 2, 4 ou 6 fois/jour pendant 1 mois (soit 50 à 250mg/jour).

Les exemples qui suivent illustrent l'invention mais ne sont pas limitatifs.

### Exemple 1 : préparation d'un extrait hydrosoluble de sucres d'avocat

50 kg d'avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire.

Les 4 kg de fruits délipidés (tourteau) sont alors broyés à froid puis extraits à reflux, en présence de 25 litres d'éthanol. La poudre épuisée en lipides est alors récupérée par filtration sur Büchner et séchée à l'étuve à 50°C, pendant 5 heures.

Le tourteau est alors lavé à l'eau déminéralisée (10 litres) puis séparé par centrifugation. La fraction soluble (liquide) est reprise pour être purifiée et concentrée selon le mode opératoire suivant :
- *Déminéralisation à l'aide de résines échangeuses d'ions :* déminéralisation des heptuloses par passage sur résines OH⁻, puis sur résine H⁺.
- *Ultrafiltration sur 10 000 Da :* l'ultrafiltration est réalisée avec un système équipé de 4 membranes de seuil de coupure 10 kDa.
- *Concentration sous vide :* la concentration de l'extrait purifié est réalisée à l'aide d'un évaporateur sous vide jusqu'à l'obtention d'une matière sèche voisine de 4 %.
- *Conditionnement :* la concentration de l'extrait est ajustée à 5 % de matière sèche et on ajoute du conservateur, puis on filtre stérilement avec une membrane de 0,2 µm de seuil de coupure et on conditionne.

Le tableau 3 suivant donne la composition de l'extrait de sucres d'avocat en C7, à 5 % de matière sèche, préparé suivant le procédé décrit ci-dessus :

**Tableau 3**

| **Aspect** | Solution de couleur jaune pâle |
|---|---|
| **Critères analytiques** | |
| Matière sèche | 5% |
| pH (dilution ¼) | 7,0 |
| Absorbance à 420 nm (dilution ¼) | 0,013 |
| Absorbance à 550 nm (dilution ¼) | 0,003 |

| **Composition (%/matière sèche)** | |
|---|---|
| Saccharose | 3,0 |
| Glucose | 7,5 |
| D-mannoheptulose | 40,0 |
| Fructose | 10,6 |
| Perséitol | 28,8 |

Suivant ce même procédé, on a préparé deux autres extraits, dont la valeur du pH, l'absorbance et la teneur en sucres en C7 sont données dans le tableau 4 suivant. La teneur en sucres en C7 correspond à la somme du perséitol et du D-mannoheptulose analysée par HPLC.

**Tableau 4**

| Lot | | 1 | 2 |
|---|---|---|---|
| Matière sèche | | 5% | 5% |
| pH (dilution ¼) | | 5,9 | 5,4 |
| Absorbance (dilution ¼) | 420 nm | 0,054 | 0,076 |
| | 550 nm | 0,004 | 0,032 |
| Sucres en C7 / matière sèche | | 80,5 | 83,4 |

### Exemple 2 : Evaluation des propriétés anti-inflammatoires des sucres d'avocat (obtenus à l'exemple 1) in vitro - kératinicytes stressés avec du LPS

Nous avons évalué la capacité à moduler la réponse inflammatoire des sucres d'avocat obtenus selon l'exemple 1, ci-après dénommés AV119, dans des kératinocytes primaires humains stressés avec du LPS (lipopolysaccharide).

### Méthodologie

Les cellules ont été pré-traitées avec 0,1% d'AV119 pendant 24h et successivement avec 10µg/ml de LPS pendant 24h. Dans une autre série d'expérience, les kératinocytes ont été pré-traités avec 30µM de calpaïne et successivement avec 10µg/ml de LPS pendant 24h. La calpaïne est un inhibiteur spécifique du facteur de transcription NFκB, clé dans la cascade inflammatoire. Un contrôle de kératinocytes traités seulement avec AV 119 a été réalisé.

L'expression des ARNm des cytokines IL-6, IL-8, IL-1α, TNF-α a été analysée par PCR en temps réel et la production et le relargage des protéines ont été analysés par ELISA dans les surnageants de culture. De plus, l'expression et la production d'ICAM-1 a aussi analysée. Finalement la phosphorylation de IkB a été analysée pour mesurer l'activation du facteur de transcription NFkB.
PCR en temps réel : Les ARN totaux ont été extraits avec le kit « High Pure RNA Isolation Kit » selon les indications du fabricant (Roche Diagnostics). 1ng d'ARN ont été retro-transcrits en ADN complémentaire, ADNc, (Expand Reverse Transcriptase, Roche Diagnostics) en utilisant des amorces hexamères (Random hexamers, Roche Diagnostics), à 42°C pendant 45min, selon les instructions du fabricant. La PCR en temps reel a été réalisée en utilisant la technologie SYBR Green avec le kit "LC Fast Start DNA Master SYBR Green kit" (Roche Diagnostics) (LightCycler 2.0 Instrument). La courbe de fusion a été analysée à la fin de chaque amplification pour s'assurer de l'absence de non-spécificité des produits de réaction. La quantification repose sur la mesure des cycles seuil CT, qui sont mesurés au début de la phase exponentielle de la réaction et sur la normalisation de la courbe standard interne obtenue avec le gène de référence beta-actine.
Test ELISA pour IL-6, IL-8, IL-1α, TNF-α et de ICAM-1 : Un protocole standard a été utilise pour les tests ELISA (Phoenix Pharmaceuticals, Inc.).
Western blot : Les protéines ont été extraites des kératinocytes par homogénéisation à froid dans un tampon de lyse (50 mM HEPES pH 7.5, 150 mM NaCl, 1% glycerol, 1% Triton, 1,5 mM MgCl₂, 5 mM EGTA) supplémenté avec 20 mM de sodium pyrophosphate, 10 mM de sodium orthovanadate et 25 mM de NaF et des inhibiteurs de protéases (aprotinine, phenylmethanesulfonyl fluoride ou PMSF). Le taux de protéines a été quantifié par la méthode de Bradford. 5µg de protéines ont été déposées sur gel de 12,5 ou 7% de polyacrylamide pour une électrophorèse et transférées sur membranes de nitrocellulose. Les membranes ont été saturées toute la nuit avec 5% de lait non gras et ensuite incubées avec un anticorps polyclonal anti-IκB-α (Santa Cruz) à 1µg/ml toute la nuit à 4°C, ou avec un anticorps monoclonal anti-phospho-IκB-α (Stressgen, Milan, Italy) à 1µg/ml pendant 2h à température ambiante. Après rinçage, les membranes ont été révélées par le système peroxydase/Chimiluminescence (ECL system, Amersham Biosciences Biotech, Milan, Italy).

### Résultats

**Tableau 5 : Expression génique (ARNm, PCR en temps réel) des cytokines pro-inflammatoires IL-6, IL-8, IL-1α, TNF-α dans des keratinocytes stréssés avec le LPS et traités avec AV119**

| | IL-6 | IL-8 | IL-1α | TNF-α |
|---|---|---|---|---|
| AV119 0,1% | 3,0 | 1,5 | 2,0 | 1,9 |
| LPS | 54,5 | 54,1 | 71,5 | 68,0 |
| LPS+AV119 0,1% | 15,3 | 10,0 | 7,2 | 17,7 |
| LPS+ Calpain 1 | 5,0 | 5,0 | 6,0 | 6,5 |

**Tableau 6 : Production protéique (ELISA) des cytokines pro-inflammatoires IL-6, IL-8, IL-1α, TNF-α dans des keratinocytes stréssés avec le LPS et traités avec AV119**

| | IL-6 | IL-8 | IL-1α | TNF-α |
|---|---|---|---|---|
| contrôle | 2 | 1,5 | 2,5 | 1,9 |
| AV119 0,1% | 4 | 3,5 | 3 | 2,8 |
| LPS | 76 | 85 | 82 | 96,0 |
| LPS+AV119 0,1 % | 13 | 10 | 11 | 17,7 |
| LPS+ Calpain 1 | 9 | 8 | 5 | 6,5 |

**Tableau 7 : Expression génique (ARNm, PCR en temps réel) de la molécule d'adhésion ICAM-1 dans des keratinocytes stréssés avec le LPS et traités avec AV119**

| | ICAM-1 |
|---|---|
| AV119 1% | 1,0 |
| LPS | 53,2 |
| LPS+AV119 1% | 20,7 |
| LPS+ Calpain 1 | 7,0 |

**Tableau 8 : Production protéique (ELISA) de la molécule d'adhésion ICAM-1 dans des keratinocytes stréssés avec le LPS et traités avec AV119**

| | ICAM-1 |
|---|---|
| ctrl | 2,0 |
| AV119 1% | 4,0 |
| LPS | 85 |
| LPS+AV119 1% | 34,0 |
| LPS+ Calpain 1 | 7,0 |

Le facteur de transcription NFkB est sous forme inactive dans le cytoplasme des cellules lorsque la sous-unité IkB qui le compose est déphosphorylée. Ainsi la phosphorylation de IkB active le facteur NFkB. Le facteur NFkB activé est ensuite transloqué dans le noyau où il va interagir avec les séquences spécifiques des gènes qu'il régule et ainsi activer leur transcription. NFkB régule nombreux gènes de l'inflammation.

Dans l'expérience, le LPS induit fortement la phosphorylation d'IkB donc l'activation du facteur de transcription NFkB, alors que AV 119 inhibe de façon très nette et avec la même intensité que l'inhibiteur de NFkB, la calpaïne, la phosphorylation d'IkB. Les sucres d'avocat AV119, dans cette expérience, montrent leur capacité à inhiber le facteur de transcription NFkB.

### Conclusion

Les résultats démontrent clairement que les sucres d'avocat AV119 sont capables d'inhiber la réponse inflammatoire induite par le LPS de la même manière que l'inhibiteur de NFκB (la calpaïne) en diminuant les cytokines IL-6, IL-8, IL-1α, TNF-α au niveau des ARNm (tableau 5) et des protéines (tableau 6). Par ailleurs, les sucres AV119 sont également capables d'inhiber la molécule d'adhésion ICAM-1 au niveau des ARNm (tableau 7) et de la protéine (tabelau 8).

De plus, les résultats montrent que le LPS est capable d'induire l'activation d'IκB en le phosphorylant alors que les sucres d'avocat tout comme l'inhibiteur spécifique de NFκB (la calpaïne) sont capables d'inhiber la phosphorylation d'IκB et donc son activation (Figure 1). Ainsi en limitant l'activation d'IκB, les sucres d'avocat limitent l'activation du facteur de transcription NFκB et inhibent ainsi l'activation de la réponse inflammatoire.

### Exemple 3 : Evaluation des propriétés anti-inflammatoires des sucres d'avocat (obtenus à l'exemple 1) in vitro - kératinicytes stressés avec du PMA

Nous avons évalué la capacité à moduler la réponse inflammatoire des sucres d'avocat obtenus selon l'exemple 1, ci-après dénommés AV119, dans des kératinocytes primaires humains stressés avec du PMA (phorbol myristic acetate).

### Méthodologie

A J0, les kératinocytes humains épidermiques normaux, notés NHEK, sont ensemencés dans du milieu KGM2 sans hydrocortisone.

A sub- confluence (à J1), les NHEK sont rincés avec du PBS puis pré-traités avec les sucres d'avocat AV119 à 0,005% et 0,05% ou par la Dexaméthasone (Référence positive) [Sigma, réf D4902] à 0,1 µM dans le milieu KGM2 sans hydrocortisone. 24 heures après, les NHEK sont traités toute la nuit avec du PMA (Phorbol Myristate Acétate) à 10 µg/ml.

Après incubation, les surnageants de culture sont récoltés et conservés à -80°C dans l'attente du dosage des cytokines (IL-1β, TNF-α et IL-8) par ELISA (kits R&D Systems).

Parallèlement, le nombre de cellules vivantes est déterminé par un test au Rouge Neutre.

La quantité de cytokine dosée pour chaque condition (concentration en pg/mL, ou DO₄₅₀) est ramenée au nombre de cellules vivantes en divisant par la valeur de DO₅₄₀ obtenue à l'issue du test au rouge neutre.

Les résultats sont comparés statistiquement en utilisant une analyse de variance one way Anova suivi d'un test de Tuckey.

### Résultats

**Tableau 9 : Analyse du relargage d'IL1β par des kératinocytes stressés avec du PMA et traités avec AV119**

| **Conditions** | **IL1β pg/ml/DO₄₅₀ Moyenne ± Ecart type** | **Significativité** | **% / rapport au contrôle** |
|---|---|---|---|
| Contrôle sans PMA | 0,729 ± 0,440 | | |
| PMA | 10,930 ± 0,190 | $$$ | 1399 |
| Dexaméthasone | 3,232 ± 0,329 | *** | -70 |
| AV119 0,005% | 4,163 ± 0,723 | *** | -62 |
| AV119 0,05% | 3,169 ± 1,141 | *** | -71 |

| | | | |
|---|---|---|---|
| $$$ P<0,001 : augmentation par le PMA par rapport au contrôle non traité (sans PMA) *** p<0,001 : inhibition par la dexaméthasone ou AV119 par rapport au PMA | | | |

**Tableau 10 : Analyse du relargage de TNFα par des kératinocytes stressés avec du PMA et traités avec AV119**

| **Conditions** | **TNFα pg/ml/DC₄₅₀ Moyenne ± Ecart type** | **Significativité** | **% / rapport au contrôle** |
|---|---|---|---|
| Contrôle sans PMA | 0,049 ± 0,006 | | |
| PMA | 0,584 ± 0,079 | $$$ | 1091 |
| Dexaméthasone | 0,328 ± 0,007 | | -44 |
| AV119 0,005% | 0,311 ± 0,019 | * | -47 |
| AV119 0,05% | 0,385 ± 0,059 | | -34 |

| | | | |
|---|---|---|---|
| $$$ P<0,001 : augmentation par le PMA par rapport au contrôle non traité (sans PMA) * p<0,01: inhibition par la dexaméthasone ou AV119 par rapport au PMA | | | |

**Tableau 11 : Analyse du relargage de IL8 par des kératinocytes stressés avec du PMA et traités avec AV119**

| **Conditions** | **IL8 pg/ml/DO₄₅₀ Moyenne ± Ecart type** | **Significativité** | **% / rapport au contrôle** |
|---|---|---|---|
| Contrôle sans PMA | 0,158 ± 0,064 | | |
| PMA | 1,765 ± 0,305 | $$$ | 1017 |
| Dexaméthasone | 0,790 ± 0,089 | *** | -55 |
| AV119 0,005% | 0,739 ± 0,037 | *** | -58 |
| AV119 0,05% | 0,792 ± 0,170 | *** | -55 |

| | | | |
|---|---|---|---|
| $$$ P<0,001 : augmentation par le PMA par rapport au contrôle non traité (sans PMA) *** p<0,001 : inhibition par la dexaméthasone ou AV119 par rapport au PMA | | | |

### Conclusion

Dans cette étude, nous avons montré que le PMA induit de façon importante les médiateurs primaires de l'inflammation (IL1β et TNFα) ainsi que le médiateur secondaire, la chémokine IL-8. Nous avons clairement démontré que les sucres d'avocat AV 119 inhibent fortement et de façon comparable à la dexaméthasone, connue pour ses propriétés anti-inflammatoires, les médiateurs pro-inflammatoires.

### Exemple 4 : Exemples de formulations - applications topiques

### FLUIDE KERATINISANT

| **Matière première / Nom commercial** | | **%** |
|---|---|---|
| CETYL ALCOHOL | | De 1 à 5 % |
| SILICONE 345 | | De 1 à 5 % |
| ANTI-OXYDANT | | De 0 à 1 % |
| EAU PURIFIEE | | QSP 100 % |
| CETRIMONIUM CHLORIDE | | De 0 à 5 % |
| **SUCRES D'AVOCAT (exemple 1)** | | De 0 à 5 % |
| EXTRAIT PEPTIDIQUE DE MACA | | De 0 à 5 % |
| HYDROLYZED WHEAT PROTEIN | | De 0 à 1 % |
| CONSERVATEUR | | De 0 à 2 % |
| PARFUM | | De 0 à 1 % |
| AJUSTEUR pH | | De 0 à 1 % |

### SHAMPOOING REGULATEUR

| **Matière première / Nom commercial** | | **%** |
|---|---|---|
| EAU PURIFIÉE | | QSP 100 % |
| LAUROAMPHOACETATE | | De 5 à 20 % |
| COCOGLUCOSIDE | | De 5 à 20 % |
| DISTEARATE DE PEG 6000 | | De 1 à 5 % |
| CONSERVATEURS | | De 0 à 2 % |
| **SUCRES D'AVOCAT estérifiés (exemple 2)** | | De 0 à 5 % |
| CYCLOCERAMIDES | | De 0 à 5 % |
| ZINC PYRITHIONE | | De 0 à 1 % |
| AJUSTEUR pH | | De 0 à 1 % |
| SEQUESTRANT | | De 0 à 1 % |
| PARFUM | | De 0 à 1 % |

### SHAMPOOING ANTICHUTE

| **Matière première / Nom commercial** | | **%** |
|---|---|---|
| EAU PURIFIÉE | | QSP 100 % |
| LAUROAMPHOACETATE | | De 5 à 20 % |
| COCOGLUCOSIDE | | De 5 à 20 % |
| DISTEARATE DE PEG 6000 | | De 1 à 5 % |
| CONSERVATEURS | | De 0 à 2 % |
| **SUCRES D'AVOCAT (exemple 1)** | | De 0 à 5 % |
| OLEODISTILLAT TOURNESOL | | De 0 à 5 % |
| AJUSTEUR pH | | De 0 à 1 % |
| SEQUESTRANT | | De 0 à 1 % |
| PARFUM | | De 0 à 1 % |

### FLUIDE DEMELANT

| **Matière première / Nom commercial** | | **%** |
|---|---|---|
| CETEARYL ALCOHOL CETEARETH - 33 | | De 1 à 5 % |
| QUATERNIUM-82 | | De 0 à 2 % |
| EAU PURIFIEE | | QSP 100 % |
| HYDROLYZED WHEAT PROTEIN | | De 0 à 5 % |
| CONSERVATEURS | | De 0 à 2 % |
| AJUSTEUR pH | | De 0 à 1 % |
| PARFUM | | De 0 à 1 % |
| EXTRAIT TOTAL DE LUPIN | | De 0 à 5 % |
| **SUCRES D'AVOCAT estérifiés (exemple 2)** | | De 0 à 5 % |

### LOTION CAPILLAIRE

| **Matière première / Nom commercial** | | **%** |
|---|---|---|
| EAU PURIFIEE | | QSP 100 % |
| METHYL PROPANEDIOL | | De 5 à 20% |
| CONSERVATEUR | | De 0 à 2 % |
| AJUSTEUR pH | | De 0 à 1 % |
| PARFUM | | De 0 à 1 % |
| **SUCRES D'AVOCAT (exemple 1)** | | De 0 à 5 % |
| LUPEOL | | De 0 à 5 % |
| ETHYLHEXYL COCOATE | | De 0 à 5% |
| PEG-40 CASTOR OIL | | De 0 à 5% |

### SPRAY COIFFANT

| **Matière première / Nom commercial** | | % |
|---|---|---|
| EAU PURIFIEE | | QSP 100 % |
| SODIUM MAGNESIUM SILICATE | | De 1 à 5 % |
| ETHANOL | | De 5 à 20 % |
| AJUSTEUR pH | | De 0 à 1 % |
| PARFUM | | De 0 à 1 % |
| **SUCRES D'AVOCAT (exemple 1)** | | De 0 à 5 % |
| PVP | | De 0 à 5% |
| PEG-40 CASTOR OIL | | De 0 à 5% |

### GEL COIFFANT

| **Matière première / Nom commercial** | | **%** |
|---|---|---|
| EAU PURIFIEE | | QSP 100 % |
| CARBOMER | | De 0 à 5 % |
| SILICONE | | De 0 à 10% |
| AJUSTEUR pH | | De 0 à 2 % |
| PARFUM | | De 0 à 1 % |
| **SUCRES D'AVOCAT (exemple 1)** | | De 0 à 5 % |
| HYDROLYZED WHEAT PROTEIN | | De 0 à 5 % |
| PEG-40 CASTOR OIL | | De 0 à 5% |

### GEL FIXANT

| **Matière première / Nom commercial** | | **%** |
|---|---|---|
| EAU PURIFIEE | | QSP 100 % |
| CARBOMER | | De 0 à 5 % |
| AMP-ACRYLATES / ALLYL METHACRYLATE COPOLYMER | | De 0 à 10% |
| AJUSTEUR pH | | De 0 à 2 % |
| PARFUM | | De 0 à 1 % |
| **SUCRES D'AVOCAT (exemple 1)** | | De 0 à 5 % |
| PEG-40 CASTOR OIL | | De 0 à 5% |

### VERNIS POUR ONGLES FRAGILES ET CASSANTS

| **Matière première / Nom commercial** | | **%** |
|---|---|---|
| ACRYLATE COPOLYMER | | De 15 à 30 % |
| ETHANOL | | QSP 100% |
| ACETONE | | De 5 à 20 % |
| **SUCRES D'AVOCAT (exemple 1)** | | De 0 à 5 % |

### Exemple 5 : Exemples de formulations -Compositions pour administration par voie orale

### 1/ Composition anti-chute des cheveux sous forme de capsules molles

### A- Composition 1

- SUCRES D'AVOCAT (exemple 1) 30 mg
- Huile de pépins de courge 100 mg
- Méthionine 100 mg
- Vitamine du groupe B (B1, B2, B3, B5, B6, B9, B12), QSP 100% des AJR
- Chélate de Zn QSP 100 % des AJR
- Chélate de Fe QSP 100 % des AJR
- Cire d'abeille
- Lécithine de soja
- Gélatine alimentaire
- Glycérine

Cette composition est administrée de 2 capsules de 500 mg par jour.

### B- Composition 2

- SUCRES D'AVOCAT estérifiés (exemple 2) 40 mg
- Huile de céréale riche en céramides et lipides polaires 300 mg
- Huile de lupin 50 mg
- Vitamine E QSP 100 % de l'AJR
- Vitamine C QSP 50 % de l'AJR
- Cire d'abeille
- Lécithine de soja
- Gélatine alimentaire
- Glycérine

Cette composition est administrée de 4 à 6 capsules de 500 mg par jour.

### 2/ Comprimés fortifiant des cheveux

- SUCRES D'AVOCAT (exemple 1) 40 mg
- Extraits de céréales (blé, sarrasin, millet, épeautre) riche en acides aminés soufrés 200 mg
- Vitamine C QSP 100 % des AJR
- Glycosaminoglycanes issus de cartilages de poissons 200 mg
- Glucidex IT 19 (agent de compression) QSP 1 comprimé de 800 mg.

Cette composition est administrée de 2 à 6 comprimés par jour.

### 3/ Formule fortifiante des ongles

- SUCRES D'AVOCAT (exemple 1) 150 mg
- Extraits de céréales (blé, sarrasin, millet, épeautre) riche en acides aminés soufrés 200 mg
- Zn sous forme de chélate
- Extrait de bambou riche en acide silicique 100 mg
- Glycosaminoglycanes issus de cartilages de poissons 200 mg
- Arôme fruit (agrume, fruit rouge), acésulfame de potassium, Glucidex IT 19 (agent de compression) QSP 1 comprimé de 2000 mg. Cette composition est administrée 1 fois par jour.

### 4/ Stick poudre anti-chute des cheveux

- SUCRES D'AVOCAT (exemple 1) 100 mg
- Extrait de thé riche en polyphénols 100 mg
- Extrait de raisin riche en OPC 50 mg
- Extrait de houblon riche en 8 PrenylNaringénine 50 mg
- Gomme xanthane
- ascorbate de sodium
- maltodextrine

Cette composition est administrée 2 fois par jour.

### 5/ Barre céréalière goût chocolat renforcement des ongles

- SUCRES D'AVOCAT (exemple 1) 150 mg
- Glycosaminoglycanes 100 mg,
- Acide hyaluronique 500 mg
- Extrait de prêle des champs riche en acide silicique 100 mg
- Tocophérols naturels 4 mg.
- Chocolat noir, oligofructose, sucre, sirop de fructose, cacao maigre en poudre, céréales croustillantes, lait écrémé en poudre, amandes, glycérol, sorbitol, huiles végétales, sirop de glucose, arôme, lait condensé sucré, lécithine de soja, mono-et diglycérides d'acides gras, sirop caramélisé, maltodextrine, sel, sorbate de potassium, alpha tocophérol. QSP une barre de 50 g.

Cette composition est administrée une fois par jour.

### 6/ Barre céréalière goût vanille anti-chute des cheveux

- SUCRES D'AVOCAT estérifiés (exemple 2) 150 mg
- Extraits de céréales (blé, sarrasin, millet, épautre) riche en acides aminés soufrés 200 mg
- Glycosaminoglycanes issus de cartilages de poissons 500 mg
- Hydrolysate de protéines riche en collagène de type II 500 mg
- Extrait de thé vert riche en polyphénols 200 mg
- Oligofructose, sucre, sirop de fructose, céréales croustillantes, lait écrémé en poudre, amandes, glycérol, sorbitol, huiles végétales, sirop de glucose, arôme, lait condensé sucré, lécithine de soja, mono-et diglycérides d'acides gras, sirop caramélisé, maltodextrine, sel, sorbate de potassium, alpha tocophérol. QSP une barre de 50 g.

Cette composition est administrée une fois par jour.

### 7/ Boisson lactée goût praliné dureté des ongles

- SUCRES D'AVOCAT (exemple 1) 150 mg
- Extrait de thé vert riche en polyphénols 100 mg.
- Vitamine du groupes B (B1, B2, B3, B5, B6, B9, B12) QSP 100 % des AJR.
- Zn, Mg, Se QSP 100 % des AJR.
- Acide hyaluronique 200 mg
- Extrait de bambou riche en acide silicique 200 mg
- Lait écrémé en poudre, arôme, fructose, blanc d'oeuf, noisettes, sucre, caramel, béta-carotène, gomme xanthane, aspartame, acésulfame de potassium, lécithine de soja, maltodextrine

Cette composition est administrée une fois par jour.

## Revendications

1. Composition comprenant au moins un sucre en C7 ou dérivé de formule (I) suivante 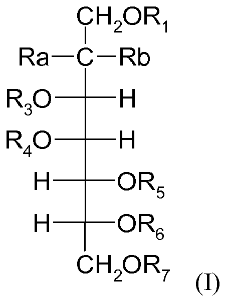 dans laquelle
Ra représente un atome d'hydrogène et Rb représente un -OR₂ ou CRaRb représente le radical CO ;
R₁, R₂, R₃, R₄, R₅, R₆ et R₇ représentent, indépendamment l'un de l'autre
- un atome d'hydrogène ou
- un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique ; ou
- un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique ;
et un excipient pharmaceutiquement acceptable pour son utilisation dans le traitement de l'alopécie.

2. Composition selon la revendication 1, **caractérisée en ce que** le degré d'estérification, pour une molécule de sucre, est compris entre 0,2 et 1.

3. Composition selon la revendication 1 ou 2, **caractérisée en ce que** le radical R représente un résidu d'un acide gras, en particulier un radical stéaryle, linoléyle, oléyle, palmityle, lauryle, myristyle, arachidyle, béhényle, lauroléyle, myristoléyle, palmitoléyle, linolényle sous ses formes α et γ, et/ou arachidonyle.

4. Composition selon l'une des revendications précédentes, **caractérisée en ce qu'**elle comprend un sucre en C7 choisi dans le groupe constitué par le mannoheptulose, le perséitol et leurs mélanges.

5. Composition selon la revendication 4, **caractérisée en ce qu'**elle comprend 0,001 à 30 % en poids de D-mannoheptulose ou de son dérivé d'acide, par rapport au poids total de ladite composition, et/ ou 0,001 à 30 % en poids de perséitol ou de son dérivé d'acide, par rapport au poids total de ladite composition.

6. Composition selon les revendications 4 ou 5, **caractérisée en ce que** la source de D-mannoheptulose et/ou de perséitol est un extrait hydrosoluble de sucres d'avocat, avantageusement susceptible d'être obtenu par un procédé comprenant les étapes successives suivantes :
- obtention d'un tourteau d'avocat, avantageusement du fruit de l'avocat, par séchage de l'avocat puis extraction des lipides ; ensuite
- délipidation complète dudit tourteau, puis lavage à l'eau ou à un milieu hydroalcoolique puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- concentration sous vide et conditionnement.

7. Composition selon la revendication 6, **caractérisée en ce que** l'extrait hydrosoluble de sucres d'avocat comprend en poids, par rapport au poids total de la matière sèche de l'extrait (composition relative déterminée par HPLC):
- D-mannoheptulose 5 à 80 %
- Perséitol 5 à 80 %
- Saccharose inférieur à 10%
- Glucose inférieur à 10%
- Fructose inférieur à 10%

8. Composition selon l'une des revendications précédentes, **caractérisée en ce qu'**elle comprend en outre un autre actif pour traiter l'alopécie, et/ou un agent anti-chute des cheveux et/ou fortifiant pour le cheveu et les ongles, et/ou un agent antipelliculaire.

9. Composition selon l'une des revendications précédentes, **caractérisée en ce qu'**elle comprend en outre un actif dermatologique choisi dans le groupe constitué par les actifs hydratants, des activateurs de la synthèse de kératine, les kératorégulateurs, les kératolytiques, les agents restructurant de la barrière cutanée, les agents cicatrisants, les agents sébo-régulateurs, les agents anti-irritants, les agents apaisants, les agents anti-inflammatoires, les agents anti-oxydants, les agents anti-âge, et leurs mélanges.

10. Composition selon l'une des revendications précédentes, **caractérisée en ce qu'**elle comprend en outre un actif choisi dans le groupe constitué par les pré et probiotiques, les agents anti-bactériens, les composés antifongiques, les conservateurs, les immunomodulateurs, les facteurs de croissance, les filtres et écrans solaires minéraux ou organiques (pigmentaires ou ultrafins)..

11. Composition selon l'une des revendications précédentes, **caractérisée en ce qu'**elle comprend en outre un actif choisi dans le groupe constitué par :
- un extrait de fruit de *Schizandra sphenanthera*, en particulier un extrait peptidique et osidique de *Schizandra sphenanthera* ;
- un extrait peptidique d'avocat ;
- une huile d'avocat ;
- des furanes d'avocat ;
- un ester gras dont la chaîne grasse est une chaîne hydrocarbonée linéaire en C7-C30 comportant entre 0 et 2 insaturations éthyléniques et pouvant être substituée par 1 à 3 groupe hydroxy et/ou 1 à 3 fonctions esters en plus de la fonction ester principale ; en particulier le 5 alpha avocuta® (butyl avocadate) ;
- des insaponifiables d'avocat et/ou de soja ;
- un oléodistillat de tournesol ;
- du lupéol ;
- des peptides de lupin ;
- un extrait total de lupin ;
- une huile de lupin ;
- un extrait peptidique de maca ;
- une oxazoline, en particulier la 2-undécyl-4,4-diméthyl-1,3-oxazoline (Cyclocéramide®) ;
- un extrait de quinoa, en particulier un extrait peptidique de quinoa ;
- des peptides de riz ;
- du beurre de Cupuaçu ;
- un concentrat de colza ou de maïs.

12. Composition selon l'une des revendications précédentes, **caractérisée en ce qu'**elle est destinée à une administration topique ou par voie orale.

13. Procédé de traitement cosmétique des phanères, en particulier des cheveux, destiné à stimuler leur croissance et/ou freiner leur chute, **caractérisé en ce qu'**il consiste à administrer une composition cosmétique comprenant au moins un dérivé de formule (I) tel que défini selon l'une quelconque des revendications 1 à 7.

14. Procédé de soin cosmétique selon la revendication 13, **caractérisé en ce que** la composition cosmétique est appliquée sur les cheveux et/ou cils et/ou ongles puis laissée au contact des cheveux et/ou cils et/ou ongles et éventuellement rincée.

15. Procédé de soin cosmétique selon la revendication 13, **caractérisé en ce que** la composition cosmétique est administrée par voie orale, de préférence sous la forme de capsules, de gélules, de comprimés, de barres céréalières ou de boissons.

## Patentansprüche

1. Zusammensetzung, umfassend mindestens einen C7-Zucker oder ein Derivat davon der folgenden Formel (I) 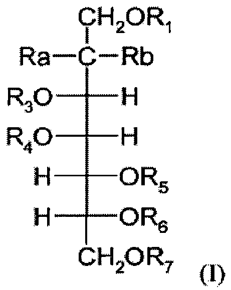 in welcher
Ra für ein Wasserstoffatom steht und Rb für ein -OR₂ steht oder CRaRb für den Rest CO steht;
R₁, R₂, R₃, R₄, R₅, R₆ und R₇ unabhängig voneinander für
- ein Wasserstoffatom oder
- einen Rest -(CO)-R, in welchem R für eine gesättigte oder ungesättigte Kohlenwasserstoffkette, welche 11 bis 24 Kohlenstoffatome enthält, welche gegebenenfalls durch einen oder mehrere Substituenten ausgewählt aus der Gruppe, die aus den Hydroxyresten (-OH), den Ethoxyresten (-OC₂H₅) und der Gruppe - SO₃M, worin M für ein Wasserstoffatom, ein Ammoniumion NH₄⁺ oder ein Metallion steht, gebildet wird, substituiert ist, steht; oder
- einen Rest -(CO)-R', in welchem R' für eine gesättigte oder ungesättigte Kohlenwasserstoffkette, welche 2 bis 10 Kohlenstoffatome enthält, welche gegebenenfalls durch einen oder mehrere Substituenten ausgewählt aus der Gruppe, die aus den Hydroxyresten (-OH), den Ethoxyresten (-OC₂H₅) und der Gruppe - SO₃M, worin M für ein Wasserstoffatom, ein Ammoniumion NH₄⁺ oder ein Metallion steht, gebildet wird, substituiert ist, steht;
stehen;
und ein pharmazeutisch annehmbares Vehikel für deren Verwendung bei der Behandlung von Alopezie.

2. Zusammensetzung nach Anspruch 1, **dadurch gekennzeichnet, dass** der Veresterungsgrad für ein Zuckermolekül zwischen 0,2 und 1 eingeschlossen liegt.

3. Zusammensetzung nach Anspruch 1 oder 2, **dadurch gekennzeichnet, dass** der Rest R für einen Rest einer Fettsäure, insbesondere einen Stearyl-, Linoleyl-, Oleyl-, Palmityl-, Lauryl-, Myristyl-, Arachidyl-, Behenyl-, Lauroleyl-, Myristoleyl-, Palmitoleylrest, einen Linolenylrest in dessen α- und γ-Formen und/oder einen Arachidonylrest steht.

4. Zusammensetzung nach einem der vorangegangenen Ansprüche, **dadurch gekennzeichnet, dass** sie einen C7-Zucker ausgewählt aus der Gruppe, die aus Mannoheptulose, Perseitol und deren Mischungen gebildet wird, umfasst.

5. Zusammensetzung nach Anspruch 4, **dadurch gekennzeichnet, dass** sie 0,001 bis 30 Gew.-% D-Mannoheptulose oder deren Säurederivat bezogen auf das Gesamtgewicht der Zusammensetzung und/oder 0,001 bis 30 Gew.-% Perseitol oder dessen Säurederivat bezogen auf das Gesamtgewicht der Zusammensetzung umfasst.

6. Zusammensetzung nach den Ansprüchen 4 oder 5, **dadurch gekennzeichnet, dass** die Quelle von D-Mannoheptulose und/oder von Perseitol ein wasserlöslicher Extrakt von Zuckern von Avocado ist, der in vorteilhafter Weise erhalten werden kann durch ein Verfahren, das die folgenden aufeinanderfolgenden Schritte umfasst;
- Gewinnung eines Ölkuchens von Avocado, vorteilhafterweise von der Frucht der Avocado, durch Trocknung der Avocado, dann Extraktion der Lipide; dann
- vollständige Delipidierung des Ölkuchens, dann Waschen mit Wasser oder einem wässrig-alkoholischen Medium, dann Dekantation und Zentrifugation, um eine an C7-Zuckern reiche lösliche Fraktion zu gewinnen (Verwerfen des Kuchens);
- Entmineralisierung der in dem vorangegangenen Schritt erhaltenen löslichen Fraktion an einem ionischen Harz; dann
- Ultrafiltration bei 10.000 Dalton; und
- Aufkonzentrierung unter Vakuum und Konditionierung.

7. Zusammensetzung nach Anspruch 6, **dadurch gekennzeichnet, dass** der wasserlösliche Extrakt von Zuckern von Avocado bezogen auf das Gewicht in Bezug auf das Gesamtgewicht der Trockensubstanz des Extrakts umfasst (relative Zusammensetzung durch HPLC bestimmt):
- D-Mannoheptulose 5 bis 80%,
- Perseitol 5 bis 80%,
- Saccharose unter 10%,
- Glucose unter 10%,
- Fructose unter 10%.

8. Zusammensetzung nach einem der vorangegangenen Ansprüche, **dadurch gekennzeichnet, dass** sie außerdem einen anderen Wirkstoff, um Alopezie zu behandeln, und/oder ein Mittel gegen das Ausfallen der Haare und/oder ein das Haar und die Nägel kräftigendes Mittel und/oder ein Antischuppenmittel umfasst.

9. Zusammensetzung nach einem der vorangegangenen Ansprüche, **dadurch gekennzeichnet, dass** sie außerdem einen dermatologischen Wirkstoff, ausgewählt aus der Gruppe, die aus den hydratisierenden Mitteln, den Aktivatoren der Keratinsynthese, den Keratoregulatoren, den Keratolytika, den Mitteln, die die Hautbarriere umstrukturieren, den die Narbenbildung fördernden Mitteln, den Talgregulatoren, den reizlindernden Mitteln, den beruhigenden Mitteln, den entzündungshemmenden Mitteln, den Antioxidationsmitteln, den Anti-Aging-Mitteln und deren Mischungen, umfasst.

10. Zusammensetzung nach einem der vorangegangenen Ansprüche, **dadurch gekennzeichnet, dass** sie außerdem einen Wirkstoff umfasst, der ausgewählt ist aus der Gruppe, die aus den Prä- und Probiotika, den antibakteriellen Mitteln, den Antimykotika, den Konservierungsmitteln, den Immunmodulatoren, den Wachstumsfaktoren, den mineralischen oder organischen (pigmentartigen oder ultrafeinen) Sonnenschutzfiltern und Sonnenschutzmitteln gebildet wird.

11. Zusammensetzung nach einem der vorangegangenen Ansprüche, **dadurch gekennzeichnet, dass** sie außerdem einen Wirkstoff umfasst, der ausgewählt ist aus der Gruppe, die gebildet wird aus:
- einem Extrakt der Frucht von *Schizandra sphenanthera*, insbesondere einen peptidischen und osidischen Extrakt von *Schizandra sphenanthera*;
- einem peptidischen Extrakt von Avocado;
- einem Avocadoöl;
- Furanen von Avocado;
- einem Fettsäureester, dessen Fettkette eine lineare C7-C30-Kohlenwasserstoffkette, welche zwischen 0 und 2 ethylenische Ungesättigtheiten umfasst und durch 1 bis 3 Hydroxygruppen und/oder 1 bis 3 Esterfunktionen zusätzlich zu der hauptsächlichen Esterfunktion substituiert sein kann, ist, insbesondere 5 alpha avocuta® (Butylavocadat);
- unverseifbaren Anteilen von Avocado und/oder von Soja;
- einem Öldestillat von Sonnenblume;
- Lupeol;
- Peptiden von Lupine;
- einem Gesamtextrakt von Lupine;
- einem Lupinenöl;
- einem peptidischen Extrakt von Maca;
- einem Oxazolin, insbesondere 2-Undecyl-4,4-dimethyl-1,3-oxazolin (Cycloceramide®);
- einem Extrakt von Quinoa, insbesondere einem peptidischen Extrakt von Quinoa;
- Peptiden von Reis;
- Cupuaçu-Butter;
- einem Konzentrat von Raps oder von Mais.

12. Zusammensetzung nach einem der vorangegangenen Ansprüche, **dadurch gekennzeichnet, dass** sie für eine topische Verabreichung oder eine Verabreichung auf oralem Wege bestimmt ist.

13. Verfahren zur kosmetischen Behandlung der Hautanhangsgebilde, insbesondere der Haare, welches dazu bestimmt ist, deren Wachstum zu stimulieren und/oder deren Ausfallen zu hemmen, **dadurch gekennzeichnet, dass** es darin besteht, eine kosmetische Zusammensetzung, die mindestens ein Derivat der Formel (I) umfasst, wie gemäß einem der Ansprüche 1 bis 7 definiert, zu verabreichen.

14. Kosmetisches Pflegeverfahren nach Anspruch 13, **dadurch gekennzeichnet, dass** die kosmetische Zusammensetzung auf die Haare und/oder Wimpern und/oder Nägel aufgetragen wird, dann in Kontakt mit den Haaren und/oder Wimpern und/oder Nägeln gelassen wird und gegebenenfalls abgespült wird.

15. Kosmetisches Pflegeverfahren nach Anspruch 13, **dadurch gekennzeichnet, dass** die kosmetische Zusammensetzung auf oralem Wege, vorzugsweise in Form von Kapseln, Hartkapseln, Tabletten, Cerealienriegeln oder Getränken, verabreicht wird.

## Claims

1. A composition containing at least one C7 sugar or derivative of following formula (I): 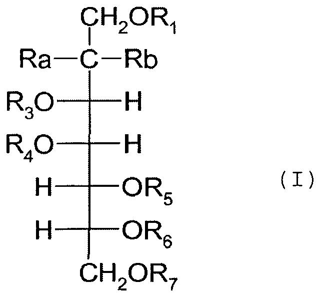 wherein:
Ra represents a hydrogen atom and Rb represents -OR₂ or CRaRb represents a CO radical;
R₁, R₂, R₃, R₄, R₅, R₆ and R₇ represent, independently of one another:
- a hydrogen atom or
- a -(CO)-R radical wherein R represents a saturated or unsaturated hydrocarbon chain containing from 11 to 24 carbon atoms, optionally substituted by one or more substituents selected from the group comprising hydroxy radicals (-OH), ethoxy radicals (-OC₂H₅) and an -SO₃M group with M representing a hydrogen atom, an ammonium ion (NH₄⁺) or a metal ion; or
- a -(CO)-R' radical wherein R' represents a saturated or unsaturated hydrocarbon chain containing from 2 to 10 carbon atoms, optionally substituted by one or more substituents selected from the group comprising hydroxy radicals (-OH), ethoxy radicals (-OC₂H₅) and an -SO₃M group with M representing a hydrogen atom, an ammonium ion (NH₄⁺) or a metal ion;
and an pharmaceutically acceptable excipient to be used to treat alopecia.

2. The composition of claim 1, wherein the degree of esterification, for a sugar molecule, is between 0.2 and 1.

3. The composition of claim 1 or claim 2, wherein the radical R represents a fatty acid residue, in particular a stearyl, linoleyl, oleyl, palmityl, lauryl, myristyl, arachidyl, behenyl, lauroleyl, myristoleyl, palmitoleyl, linolenyl in its α and γ forms, and/or arachidonyl radical.

4. The composition of one of the preceding claims, wherein said composition contains a C7 sugar selected from the group comprising mannoheptulose, perseitol and mixtures thereof.

5. The composition of claim 4, wherein said composition contains 0.001 to 30% by weight D-mannoheptulose or an acid derivative thereof, in relation to the total weight of said composition, and/or 0.001 to 30% by weight perseitol or an acid derivative thereof, in relation to the total weight of said composition.

6. The composition of claim 4 or claim 5, wherein the source of D-mannoheptulose and/or perseitol is an avocado sugar water-soluble extract, advantageously obtained by a method comprising the following successive steps:
- an avocado oil cake is obtained, advantageously from avocado fruit, through drying and extraction of the lipids; after which
- cryogrinding and total delipidation of said oil cake, then decanting and centrifugation in order to recover a soluble fraction rich in C7 sugars (elimination of the cake);
- demineralization on ionic resin of said soluble fraction obtained in the preceding step; then
- ultrafiltration at 10,000 daltons; and
- concentration under vacuum and packaging.

7. The composition of claim 6, wherein the avocado sugar water-soluble extract contains by weight in relation to the total weight of the dry matter of the extract (relative composition determined by HPLC):
- D-Mannoheptulose 5-80%
- Perseitol 5-80%
- Sucrose less than 10%
- Glucose less than 10%
- Fructose less than 10%

8. The composition of one of the preceding claims, wherein said composition further contains another active agent for treating alopecia, and/or an anti-hair loss and/or hair and nail strengthening agent, and/or an anti-dandruff agent.

9. The composition of one of the preceding claims, wherein said composition further contains a dermatological active agent selected from the group comprising moisturizing active agents, keratin synthesis activators, keratoregulators, keratolytics, agents that repair the cutaneous barrier, healing agents, sebum-regulating agents, anti-irritant agents, soothing agents, anti-inflammatory agents, antioxidant agents, anti-aging agents, and mixtures thereof.

10. The composition of one of the preceding claims, wherein said composition further contains an active agent selected from the group comprising prebiotics and probiotics, antibacterial agents, antifungal compounds, preservatives, immunomodulators, growth factors and inorganic or organic sun filters and screens (pigmentary or ultrafine).

11. The composition of one of the preceding claims, wherein said composition further contains an active agent selected from the group comprising:
- a *Schisandra sphenanthera* fruit extract, in particular a *Schisandra sphenanthera* peptide and sugar extract;
- an avocado peptide extract;
- an avocado oil;
- avocado furans;
- a fatty ester whose fatty chain is a linear C₇-C₃₀ hydrocarbon chain comprising between 0 and 2 ethylene unsaturations and which can be substituted by 1 to 3 hydroxy groups and/or 1 to 3 ester functional groups in addition to the principal ester functional group, in particular 5-α Avocuta^{®} (butyl avocadate);
- avocado and/or soya unsaponifiables;
- a sunflower oleodistillate;
- lupeol;
- lupin peptides;
- a total lupin extract;
- a lupin oil;
- a maca peptide extract;
- an oxazoline, in particular 2-undecyl-4,4-dimethyl-1,3-oxazoline (Cycloceramide^{®});
- a quinoa extract, in particular a quinoa peptide extract;
- rice peptides;
- cupuagu butter;
- a rapeseed or corn concentrate.

12. The composition of one of the preceding claims, wherein said composition is intended for topical or oral administration.

13. A method of cosmetic treatment of the hair and nails, in particular the hair, intended to stimulate the growth thereof and/or to slow the loss thereof, wherein said method consists in administering a cosmetic composition containing at least one derivative of formula (I) such as defined according to any one of claims 1 to 7.

14. The method of cosmetic care of claim 13, wherein the cosmetic composition is applied to the hair and/or eyelashes and/or nails and then left in contact with the hair and/or eyelashes and/or nails and optionally rinsed.

15. The method of cosmetic care of claim 13, wherein the cosmetic composition is administered orally, preferably in the form of soft or hard capsules, tablets, cereal bars or beverages.
